(19) Europäisches Patentamt
European Patent Office
Office européen des brevets

(11) Publication number: **0 241 021**
**A2**

(12) # EUROPEAN PATENT APPLICATION

(21) Application number: **87105229.6**

(22) Date of filing: **08.04.87**

(51) Int. Cl.⁴: **C12N 15/00**, C12P 21/02

(30) Priority: **08.04.86 JP 79086/86**

(43) Date of publication of application:
**14.10.87 Bulletin 87/42**

(84) Designated Contracting States:
**CH DE ES FR GB LI**

(71) Applicant: **THE GREEN CROSS CORPORATION**
**15-1, Imabashi-1-chome Higashi-ku**
**Osaka-shi**
**Osaka 541(JP)**

(72) Inventor: **Tsujikawa, Muneo**
**21-301, Kita Funabashi-cho**
**Hirakata-shi Osaka(JP)**
Inventor: **Kobayashi, Kaoru**
**2-4-5-304, Honmachi**
**Toyonaka-shi Osaka(JP)**
Inventor: **Kawabe, Haruhide**
**29-14, Yamada Nishi 3-chome**
**Suita-shi Osaka(JP)**
Inventor: **Arimura, Hirofumi**
**18-1-401, Uenozaka 2-chome**
**Toyonaka-shi Osaka(JP)**
Inventor: **Nishida, Masayuki**
**12-1, Kayogaoka 2-chome**
**Nagaokakyo-shi Kyoto(JP)**
Inventor: **Suyama, Tadakazu**
**3-7, Matsuigaoka 4-chome Tanabe-cho**
**Tsuzuki-gun Kyoto(JP)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) **Method of producing HBsAg containing amino acid sequence encoded by the late pre S region of HB virus and the said HBsAg.**

(57) Pre S-HBsAg (HBsAg containing the amino acid sequence encoded by a late part of the Pre S region of HB virus) is produced by using transformed cells derived from CHO cells or normal liver cells as host cells with the SV40 or HBV promoter as a promoter and the SV40 or HBV enhancer as an enhancer.

EP 0 241 021 A2

# METHOD OF PRODUCING HBsAg CONTAINING AMINO ACID SEQUENCE ENCODED BY THE LATE Pre S REGION OF HB VIRUS AND THE SAID HBsAg

## FIELD OF THE INVENTION

The present invention relates to a method of producing Pre S-HBsAg proteins in mammalian cells transformed with recombinant DNA suited for the transformation of mammalian cells and containing the hepatitis B virus surface antigen (HBsAg) gene inclusive of a late part of the Pre S region (Pre S) of hepatitis B virus.

## BACKGROUND OF THE INVENTION

Hepatitis B virus (HBV) is virus capable of infecting the human liver to cause hepatitis, liver cirrhosis or liver cancer in the host. The most efficient means of preventing HBV infection is the HBsAg vaccine. However, attempts to infect cultured cells with HBV have been unsuccessful. As a result, purified HBsAg from human serum is mainly used as the vaccine material.

The human serum-derived HBsAg particles are mainly composed of the peptide P24 (molecular weight 24,000) and the peptide P27 (molecular weight 27,000), which is glycosylated form of P24. In addition, they contain human serum-derived HBsAg particles peptide P31 (molecular weight 31,000) and peptide P34 (molecular weight 34,000).

Machida et al, Gastroenterology, 85:268-274 (1983), report that a site capable of binding polymerized human serum albumin (pHSA), namely a pHSA receptor, is present on peptide P31 and peptide P34 and propose the hypothesis that HBV shows hepatotropism.

Recently, attempts have been made to obtain expression of HBsAg in yeast or mammalian cells using recombinant DNA technology.

In the case of yeast expression of HBsAg particles was successfully achieved using the promoter of glutaraldehyde-3-phosphate dehydrogenase gene (Nature, 298, 347-350 (1982)). On the other hand, expression of HBsAg particles in mammalian cells was achieved in mouse C127 using bovine papiloma virus vector (Gene, 32, 357-368 (1984)) and in mouse L cells by introducing therein a plasmid containing HBV gene in tandem fashion (Proc. Natl. Acad. Sci. U.S.A., 77, (8), 4549-4553 (1980)).

On the HBV genome, there exists the so-called Pre S region on the 5′ side of the P24 encoding HBsAg region. The Pre S region contains two or three translation initiation codons (ATG) depending on the subtype. The peptide portion, comprising 55 amino acid residues, encoded by the region from the most downstream ATG codon to the codon immediately upstream from the HBsAg gene, bears the receptor for pHSA (Machida et al, Gastroenterology, 86:910-918 (1984)). Therefore, it is considered that those HBsAg species which include the Pre S product might possibly be more effective as infection-preventing vaccines than those HBsAg species which comprise P24 and P27. In fact, it is reported that those HBsAg particles which are higher in the Pre S product content show higher immunogenicity in mice (Coursaget et al, Lancet, i:1152-1153 (1985)).

The DNA sequence of Pre S is known and plasmids carrying Pre S are also known ( Molecular Cloning, Cold Spring Harbor Laboratory (1982)). For example, the sequence shown below is known as the full length DNA sequence of Pre S. In the examples of present invention, the plasmid pPre S (Fig.1) commercially available from Biogen was employed. The use of the entire length of the Pre S region is not always necessary. The fragment resulting from cleavage at the second or third ATG site is sufficient for the purpose of the invention and in fact is preferable.

DNA and Amino Acid Sequence of Pre S

```
  1
  Met Gly Gln Asn Leu Ser Thr Ser Asn
  ATG GGG CAG AAT CTT TCC ACC AGC AAT

10
Pro Leu Gly Phe Phe Pro Asp His Gln Leu
CCT CTG GGA TTC TTT CCC GAC CAC CAG TTG

20
Asp Pro Ala Phe Arg Ala Asn Thr Asn Asn
GAT CCA GCC TTC AGA GCA AAC ACC AAC AAT

30
Pro Asp Trp Asp Phe Asn Pro Asn Lys Asp
CCA GAT TGG GAC TTC AAT CCC AAC AAG GAC

40
Thr Trp Pro Asp Ala Asn Lys Val Gly Ala
ACC TGG CCA GAC GCC AAC AAG GTA GGA ACT

50
Gly Ala Phe Gly Leu Gly Phe Thr Pro Pro
GGA GCA TTC GGG CTA GGG TTC ACC CCA CCG

60
His Gly Cly Leu Leu Gly Trp Ser Pro Gln
CAC GGA GGC CTT TTG GGG TGG AGC CCT CAG

70
Aka Gln Gly IIe Met Gln Thr Leu Pro Ala
GCT CAG GGC ATA ATG CAA ACC TTG CCA GCA

80
Asn Pro Pro Pro Ala Ser Thr Asn Arg Gln
AAT CCG CCT CCT GCC TCT ACC AAT CGC CAG

90
Ser Gly Arg Arg Pro Thr Pro Leu Ser Pro
TCA GGA CGG CAG CCT ACC CCG CTG TCT CCA

100
Pro Leu Arg Thr Thr Hls Pro Gln Ala
CCT CTG AGA ACC ACT CAT CCT CAG GCC

    110
Met His Trp Asn Ser Thr Thr Phe His Gln
ATG CAC TGG AAC TCC ACA ACC TTC CAC CAA

    120
Thr Leu Gln Asp Pro Arg Val Arg Gly Leu
ACT CTG CAA GAT CCC AGA GTR AGA GGC CIG

    130
Tyr Phe Pro Ala Gly Gly Ser Ser Ser Gly
TAT TCC CCT GCT GGT GGC TCC AGT TCA GGG

    140
Thr Val Asn Pro Val Pro Thr Thr Thr Ser
ACA GTA AAC CCT GTT CCG ACT ACT ACC TCT

    150
Pro Ile Ser Ser Ile Phe Ser Arg Ile Gly
CCC ATA TCG TAC ATC TTC TCG AGG ATT GGG

    160
Asp Pro Ala Leu Asn
GAC CCT GCG CTG AAC
```

The DNA sequence of th HBsAg gene is also known and plasmids carrying said gene are known. The Pre S-HBsAg gene has the restriction enzyme map shown in Fig. 2. In the examples of the present invention, the plasmid pPre S (Fig. 1) commercially available from Biogen was used.

While HBsAg includes the subtypes adw (Valenzucala et al, Nature, 280:815-819 (1979), ayw (Charnay et al, Proc. Natl. Acad. Sci. U.S.A., 76:2222-2226 (1979), ad/yw (Pasek et al, Nature, 282:575-579 (1979), etc., HBsAg subtype ad/yw was employed in the example of the present invention. HBsAg subtype ad/yw is preferred since it has both of the allelic antigen determinants a and y.

## SUMMARY OF THE INVENTION

The present invention has been completed based on the finding that novel recombinant DNAs constructed by joining an appropriate promoter and an appropriate enhancer to that HBV DNA which comprises the HBsAg gene as well as the minimum unit contained in the Pre S gene region which is necessary for the pHSA binding activity, namely that part of the Pre S region which begins with the third ATG in said region and reaches the HBsAg gene, at sites upstream from said HBV DNA, when used for the transformation of appropriate cells, gives rise to the productio of HBsAg particles at least comparable in immunological activity to human serum-derived HBsAg proteins.

## BRIEF DESCRIPTION OF THE DRAWINGS

Fig. 1 shows the restriction enzyme map of the plasmid pPre S used in the invention;

Fig. 2 shows the restriction enzume map of the Pre S-HBsAg gene used in the invention;

Fig. 3 shows the construction scheme for a plasmid for Pre S-HBsAg production which carries the SV40 enhancer and the SV40 promoter;

Figs. 4-6 show the construction schemes for plasmids pSV-$G_2$, pPS411 and pSV-2nd, respectively;

fig. 7 shows the restriction enzyme map of and the construction scheme for a specific example of the plasmid for Pre S-HBsAg production, pSH3;

Figs. 8 and 9 show the construction scheme for plasmids pSH1 and pSH2, respectively; and

Fig. 10 shows the restriction enzyme map of the selective plasmid pSV-NEO used for cotransfection.

In the figures, $Ap^r$ stands for ampicillin resistance gene, CIP for calf intestinal alkaline phosphatase, S-J for splicing junction, $Neo^r$ for neomycin resistance gene and SV40 poly-A for SV40 poly(A) addition signal.

## DETAILED DESCRIPTION OF THE INVENTION

The methods of producing recombinant DNAs and transformed cells according to the present invention and the production of Pre S-HBsAg proteins using said transformed cells are described in the following in more detail.

### (1) Preparation of recombinant DNAs for the expression of Pre S-HBsAg proteins

An embodiment of the expression vector to be used in the practice of the invention is pSV-$G_2$ (Fig. 3). pSV-$G_2$ was prepared from pcDV-1 and pL1 which were prepared by Okayama & Berg as a cloning vector for cDNA and obtained from Pharmacia AB (Fig.4). This vector has the SV40 enhancer, SV40 early promoter, splicing junction (S-J) and poly(A) addition signal (SV40 poly-A) and thus includes those elements which are necessary for the expression of a useful substance in mammalian cells. It is possible to bring about expression of Pre S-HBsAg by inserting the Pre S-HBsAg gene into this vector at a site between the splicing junction and the poly(A) signal in the proper reading direction and introducing the product into mammalian cells. For instance, the fragment which begins with the third ATG contained in the late Pre S region coding for pHSA binding activity and includes the HBsAg gene is excised out of the Pre S-HBsAg gene from pPS411 and joined to the expression vector-pSV$G_2$ to give pSV-3rd (Fig. 3). pPS411 was prepared from pPre S obtained from Biogen by adding Hin dIII linker at the end of the Pre S-HBsAg structural gene of pPre S and inserting the product at HindIII site of pBR322, a plasmid of E. coli (Fig.5).

pSV-2nd, which is 2nd Pre S-HBSAg expression plasmid, can also be used. This plasmid was prepared by excising out the 2nd Pre S-HBsAg gene of pPre S with BanII, adding EcoRI linker and inserting the product at the Eco RI site of pSV-$G_2$ in the appropriate reading direction (Fig. 6). The promoter-enhancer combinations found in the plasmids shown in Fig. 7, namely pSH1, pSH2 and pSH3, are suitable as well. pSH1 was prepared by deleting the SV40 enhancer, SV40 early promoter, splicing junction (S-J) and Pre S region from pSV-3rd, inserting a fragment containing the HBV promoter and Pre S region to form a plasmid pH1 and further inserting the SV40 enhancer fragment in the SspI site upstream the HBV promoter of pH1 (Fig.8). pSH2 was prepared by deleting the SV40 enhancer, SV40 early promoter and splicing junction (S-J) from pSV-1st composed of 1st Pre S-HBsAg gene connected to pSV-$G_2$, and inserting only the SV40 enhancer fragment at the BanHI site in the region of Pre S (Fig.9). Further, pSH3 was prepared by deleting the SV40 enhancer from pSV-3rd and inserting the enhancer region of HBV. Thus, the SV40 enhancer-HBV promoter, HBV enhancer-SV40 promoter and SV40 enhancer-SV40 promoter combinations are given as examples. That fragment which begins with the second ATG in the Pre S region and includes the HBsAg gene can be used in the same manner as that which begins with the third ATG.

(2) Transformation of mammalian cells

Chinese hamster ovary (CHO) cells are suitably used as the host cells. Ham's F-12 medium (Gibco) containing 10% (v/v) fetal calf serum is used as the medium. The recombinant DNA is introduced into the mammalian cells by the calcium phosphate precipitation method (Strain et al, Biochem.. J., 218:475-482 (1984)). This method comprises dropping a calcium chloride solution into a phosphate buffer containing the recombinant DNA to thereby cause formation of minute DNA-calcium phosphate precipitate particles and uptake of such precipitate particles by the cells. Those cells in which the Pre S-HBsAg gene is expressed are selected by the method of Subramani et al, Anal. Biochem.., 135:1-15 (1983) which comprises simultaneously introducing a drug resistance gene and selecting drug resistant strains. In the practice of the invention, the $Neo^r$ gene which develops the resistance to G-418 (aminoglycoside 3'-phosphotransferase II) in mammalian cells is suitably used. An example of the $Neo^r$gene expression plasmid is pSV-NEO (Fig. 10) constructed by joining the $Neo^r$ gene to a vector having the SV40 promoter, splicing junction and poly(A) addition signal. More particularly, pSV-NEO was prepared by excising out of the pNEO obtained from Pharmacia AB containing $Neo^r$ derived from transposon Tn5, adding thereto KpnI linker and inserting the product at KpnI site of pSV-G, in the proper reading direc tion. Cotransfection with pSV-3rd and pSV-NEO as used in a mole ratio of 100:1 gives high probability of uptake of pSV-3rd in G-418 resistant strains, hence a high incidence of Pre S-HBsAg protein expression. Thus, cells capable of Pre S-HBsAg protein expression can be obtained by selection from among G-418 resistant strains. Other mammalian cells suited for use are normal hepatocytes such as chang liver cells (ATCC No. CCL13) and chimp liver cells (chimpanzee liver cells; ATCC No. CCL6311).

(3) Production of Pre S-HBsAg proteins

Introduction of the recombinant DNA into mammalian cells and selection and cultivation of transformed cells give cell strains capable of releasing HBsAg into the culture fluid. HBsAg-positive cells also have pHSA-binding activity and are expected to be at least comparable in immunogenicity to human serum-derived HBsAg. Therefore, they are usable as HBV vaccines.

The following examples are further illustrative of the production of cells transformed with the recombinant DNA according to the invention and the production of Pre S-HBsAg proteins.

EXAMPLE 1

(1) Construction of the recombinant DNA pSV-3rd

A 1.2-kbp fragment ranging from the third ATG of the Pre S region to the HBsAg gene region was excised from the plasmid pPS411, which is derived from pBR322 by insertion of the entire Pre S-HBsAg gene at the HindIII site, using MstII and HindIII and inserted into the mammalian cell expression vector pSV$G_2$ at the EcoRI site in the proper reading direction to give the plasmid pSV-3rd. More specifically, pPS411 (10 µg) was treated with 12 units of MstII and 12 units of HindIII at 37°C for 5 hours and DNA was

recovered by two extractions with phenol and precipitation with ethanol. This DNA was rendered blunt-ended by treatment with 10 units of DNA polymerase I Klenow fragment in the presence of 40 mM Tris-hydrochloride buffer (pH 7.2), 8 mM magnesium sulfate, 80 µm dithiothreitol and 2 mM dNTP at room temperature for 30 minutes and then dephosphorylated at the 5′ end by treatment with 1 unit of calf intestinal alkaline phosphatase in 50 mM Tris-hydrochloride buffer (pHm 8.2) at 37°C for 1 hour. Then, DNA was recovered by two chloroform-phenol extractions and precipitation with ethanol. The EcoRI linker pGGAATTCC was joined to 4 µg of this DNA by overnight treatment at 16°C with 5.6 units of T4 DNA ligase in the presence of 66 mM Tris-hydrochloride buffer (pH 7.6), 6.6 mM magnesium chloride, 10 mM dithiothreitol, 1.0 mM ATP and 2 µg of said EcoRI linker. The resulting DNA was recovered by precipitation with ethanol, digested with 50 units of EcoRI at 37°C for 5 hours and then subjected to 0.8% low melting point gel electrophoresis. A 1.2-kbp DNA fragment was recovered by two extractions with phenol from the corresponding gel section and the subsequent precipitation with ethanol.

The 1.2 kbp Pre S-HBsAg fragment with the EcoRI linker joined thereto was inserted into the mammalian cell expression vector $pSVG_2$, which has the SV40 enhancer, SV40 promoter, splicing junction (S-J) and SV40 poly(A) addition signal, at the EcoRI site. More specifically, 3µg of $pSVG_2$ was digested with 10 units of EcoRI at 37°C for 6 hours and then dephosphorylated at the 5′ end by treatment with 20 units of calf intestinal alkaline phosphatase in 50 mM Trishydrochloride buffer (pH 8.2) at 37°C for 1 hour.

The reaction product was subjected to 0.8% low melting point gel electrophoresis. The desired DNA fragment was recovered from the corresponding gel portion by two extractions with phenol and precipitation with ethanol. The EcoRI-digested $pSVG_2$ (1 µg) and the 1.2-kbp Pre S-HBsAg fragment (0.5 µg) were ligated together by overnight treatment with 8.4 units of T4 DNA ligase at 16°C in a reaction mixture containing 66 mM Tris-hydrochloride buffer (ph 7.6), 6.6 mM magnesium chloride, 10 mM dithiothreitol and 1.0 mM ATP. The ligation product was used to transform Escherichia coli HB101 to give ampicillin-resistant transformants. pSV-3rd with the Pre S-HBsAg fragment inserted in the proper reading direction was recovered from among the transformants by analyzing the EcoRI, BamHI and SalI + XbaI cleavage patterns.

(2) Transformation of mammalian cells

CHO cells were transformed by the calcium phosphate precipitation method. More specifically, 2.5 ml of twice concentrated HEPES buffer (2 x HBS) (10 g/liter HEPES (N-(2-hydroxyethyl)piperazine-N′-2-ethanesulfonic acid), 6 g/liter NaCl, adjusted to pH 7.1, 50 µl of an aqueous solution containing 70 mM $NaH_2HPO_4$ and 70 mM $Na_2HPO_4$, 100 µl of a 1 µg/ml salmon sperm DNA solution and 100 µl of a recombinant DNA solution containing 10 µg of pSV-3rd and 0.1 µg of pSV-NEO (Fig. 2) were placed in a tube and the volume was made to 4.7 ml with sterile water to give solution B. To this solution B was added dropwise 300 µl of solution A (2 M $CaCl_2$) slowly with aeration and stirring of solution B. After completion of the dropping of solution A, aeration was continued for 1-2 minutes. The resulting homogeneous precipitate was allowed to stand for 10 minutes. CHO cells (5 x $10^5$ cells) were grown in a 100-mm dish the day before the transfection day and cultivated in a $CO_2$ incubator for 24 hours. Ham's F-12 medium containing 10% (v/v) fetal calf serum was used as the culture medium. A 1-ml portion of the precipitate suspension was added dropwise to the culture dish and caused to disperse uniformly. After causing the minute DNA-calcium phosphate precipitate particles to be adsorbed on the cells by allowing the mixture to stand for 10 minutes, the cells were cultivated again in a $CO_2$ incubator. Then, the medium was replaced with a fresh portion of the same medium 18 hours after transfection and the medium was replaced with G-418 containing medium after further 48 hours of cultivation. The concentration of G-418 in the medium was 400 µg/ml. Colonies grown on the G-418 containing medium were separated by the cylinder method to give G-418-resistant transformed cells.

(3) Production of Pre S-HBsAg

The cultures of the above G-418 resistant cells were examined for HBsAg activity by the RPHA method [using Antihebcel (Green Cross Corp.)] and the EIA method [using Auszyme II (Dainabot)]. HBsAg-positive strains were obtained with high frequency from among such G-418 resistant strains. The obtained cultures were further examined for pHSA-binding activity by the pHA method for pHSA (Lenkei et al, J. Immunol. Methods, 16:23-30 (1977)). pHSA-binding activity was found in HBsAg-positive cell supernatants, and the expression of the Pre S region was thus confirmed. A supernatant sample was purified by centrifugation on

a cesium chloride density gradient and subjected to molecular weight determination by SDS PAGE. Three bands corresponding to the molecular weights of about 35,000, about 27,000 and about 24,000 were confirmed. It was thus confirmed that the transformed cells had produced Pre S-HBsAg peptides. Electron microscopy revealed that the Pre S-HBsAg particles obtained in accordance with the present invention are spherical and about 22 nm in diameter.

The antigenicity of HBsAg expressed in pSV-3rd/CHO cells were determined using mouse BALB/c strain.

The results obtained are shown below.

| | Positiviy (%) | | | | |
|---|---|---|---|---|---|
| Dose (μg) | 0.5 | 1 | 2 | 4 | 8 |
| CHO sAb | 0 | 20 | 88 | 100 | 86 |
| Pre SAb | 10 | 60 | 75 | 100 | 100 |
| Plasma sAb | 63 | 80 | 94 | 97 | 100 |

While the invention has been described in detail and with reference to specific embodiments thereof, it will be apparent to one skilled in the art that various changes and modifications can be made therein without departing from the spirit and scope thereof.

## Claims

1. A method of producing HBsAg containing the amino acid sequence encoded by at least a later part of the Pre S region of HB virus, which comprises (A) transforming cells selected from the group consisting of Chinese hamster ovary cells and normal liver cells with (1) recombinant DNA comprising sequentially (1) the SV40 or HBV promoter as a promoter, (2) the SV40 or HBV enhancer as an enhancer, (3) the DNA fragment which begins from at least the third ATG contained in the Pre S region and includes the HBsAg gene, and (4) poly(A) addition signal; (B) culturing the resulting transformed cells; and (C) isolating HBsAg from the cultured medium.

2. The method of producing Pre S-HBsAg as claimed in claim 1, wherein said amino acid sequence comprises at least 55 amino acid residues starting with that methionine which is encoded by the third ATG site in said Pre S region.

3. The method of producing Pre S-HBsAg as claimed in claim 2, wherein said 55 amino acid residues constitute the following sequence:

```
Met His Trp Asn Ser Thr Thr Phe His Gln Thr
ATG CAC TGG AAC TCC ACA ACC TTC CAC CAA ACT

Leu Gln Asp Pro Arg Val Arg Gly Leu Tyr Phe
CTG CAA GAT CCC AGA GTG AGA GGC CTG TAT TTC

Pro Ala Gly Gly Ser Ser Ser Gly Thr Val Asn
CCT GCT GGT GGC TCC AGT TCA GGG ACA GTA AAC

Pro Val Pro Thr Thr Thr Ser Pro Ile Ser Ser
CCT GTT CCG ACT ACT ACC TCT CCC ATA TCG TCA

Ile Phe Ser Arg Ile Gly Asp Pro Ala Leu Asn
ATC TTC TCG AGG ATT GGG GAC CCT GCG CTG AAC
```

4. The method of producing Pre S-HBsAg as claimed in claim 1, wherein said HBsAg is of the subtype adyw.

5. The method of producing Pre S-HBsAg as claimed in claim 1, wherein the SV40 promoter, the HBV enhancer, and chang liver cells or chimp liver cells are used in combination.

6. The method of producing Pre S-HBsAg as claimed in claim 1, wherein the HBV promoter, the SV40 enhancer, and chinese hamster ovary cells or chang liver cells or chimp liver cells are used in combination.

7. The method of producing Pre S-HBsAg as claimed in claim 1, wherein the SV40 promoter, the SV40 enhancer, and chang liver cells or chimp liver cells are used in combination.

8. The method of producing Pre S-HBsAg as claimed in claim 1, wherein said DNA fragment begins from at least the second ATG contained in the Pre S region and includes the HBsAg gene.

9. HBsAg produced by the method as claimed in claim 1.

10. HBsAg produced by the method as claimed in claim 8.

Fig 1

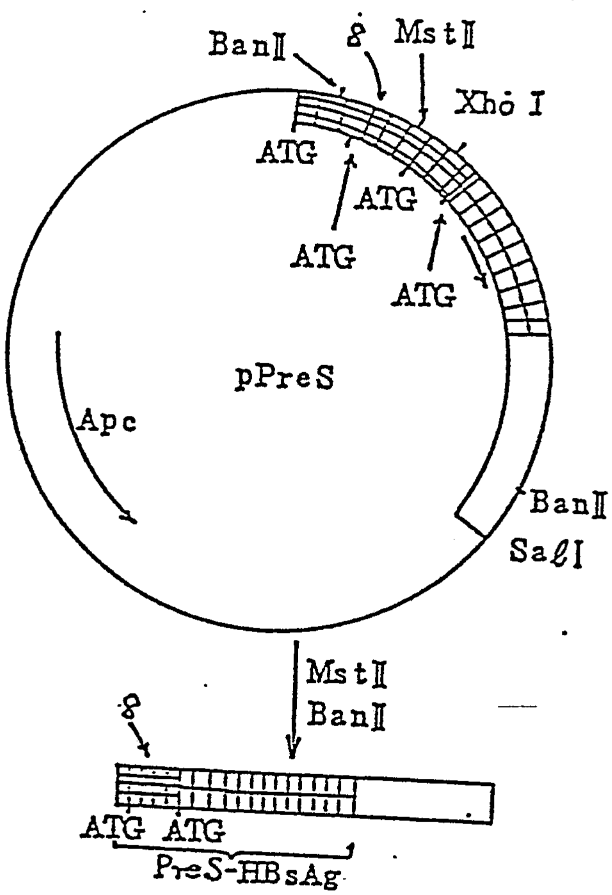
BanII
δ
MstII
XhoI
ATG
ATG
ATG
ATG
pPreS
Apc
BanII
SalI
MstII
BanII
δ
ATG ATG
PreS-HBsAg

Fig. 2

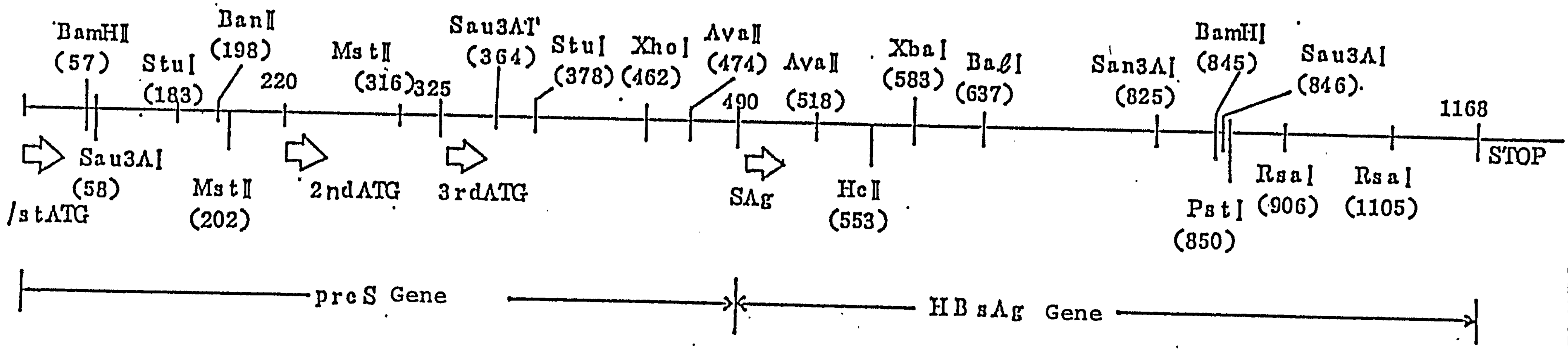
BamHII (57)
Sau3AI (58)
1stATG
StuI (183)
BanII (198)
MstII (202)
220
2ndATG
MstII (316)
325
3rdATG
Sau3AI (364)
StuI (378)
XhoI (462)
AvaII (474)
490
SAg
AvaII (518)
HcII (553)
XbaI (583)
BalI (637)
Sau3AI (825)
BamHI (845)
Sau3AI (846)
PstI (850)
RsaI (906)
RsaI (1105)
1168
STOP
preS Gene
HBsAg Gene

Fig. 3

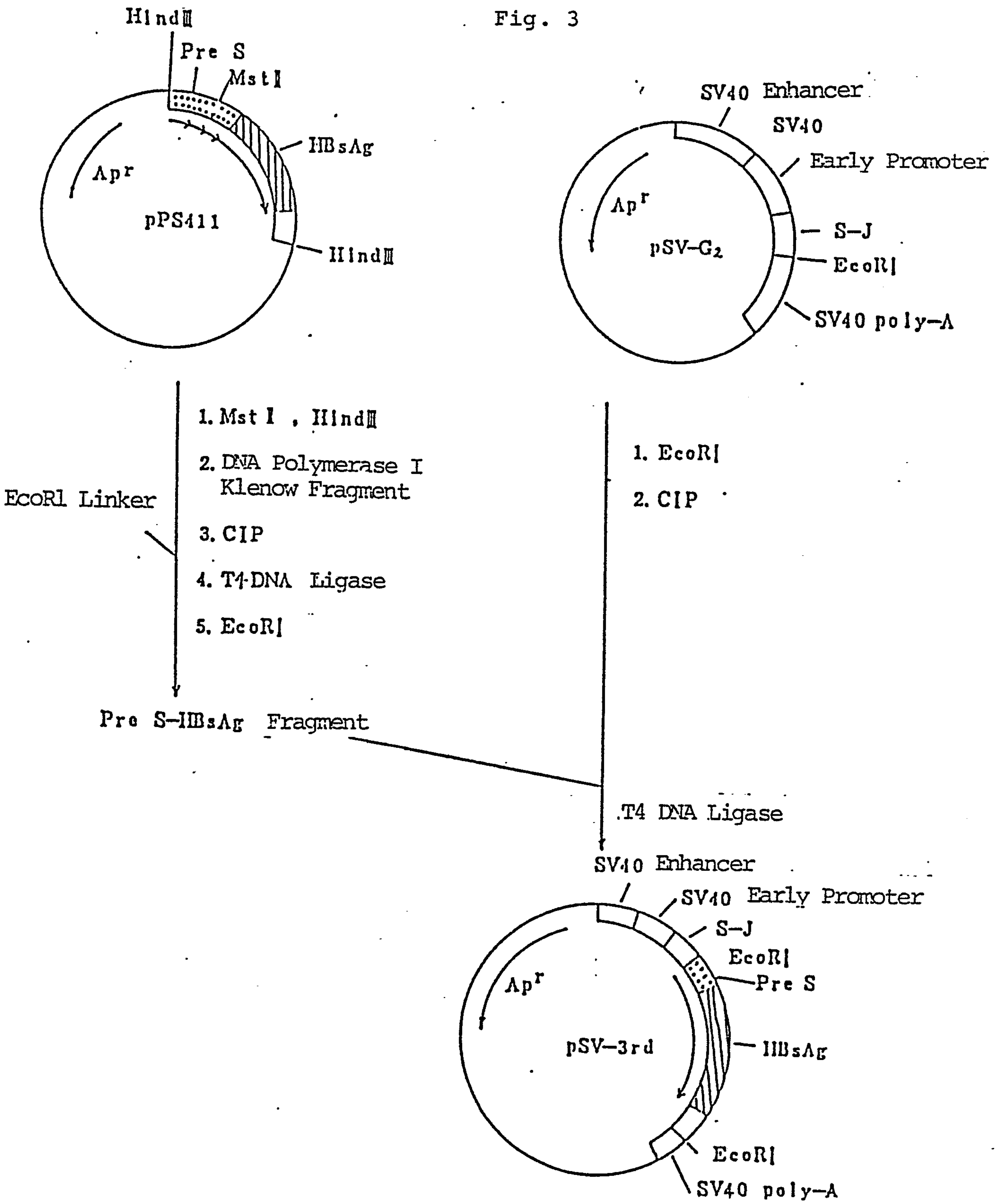

HindIII
Pre S
MstII
HBsAg
Apr
pPS411
HindIII
SV40 Enhancer
SV40
Early Promoter
Apr
pSV-G2
S-J
EcoRI
SV40 poly-A
1. MstII , HindIII
2. DNA Polymerase I
Klenow Fragment
3. CIP
4. T4-DNA Ligase
5. EcoRI
EcoRI Linker
Pre S-HBsAg Fragment
1. EcoRI
2. CIP
T4 DNA Ligase
SV40 Enhancer
SV40 Early Promoter
S-J
EcoRI
Pre S
Apr
pSV-3rd
HBsAg
EcoRI
SV40 poly-A

Fig. 4

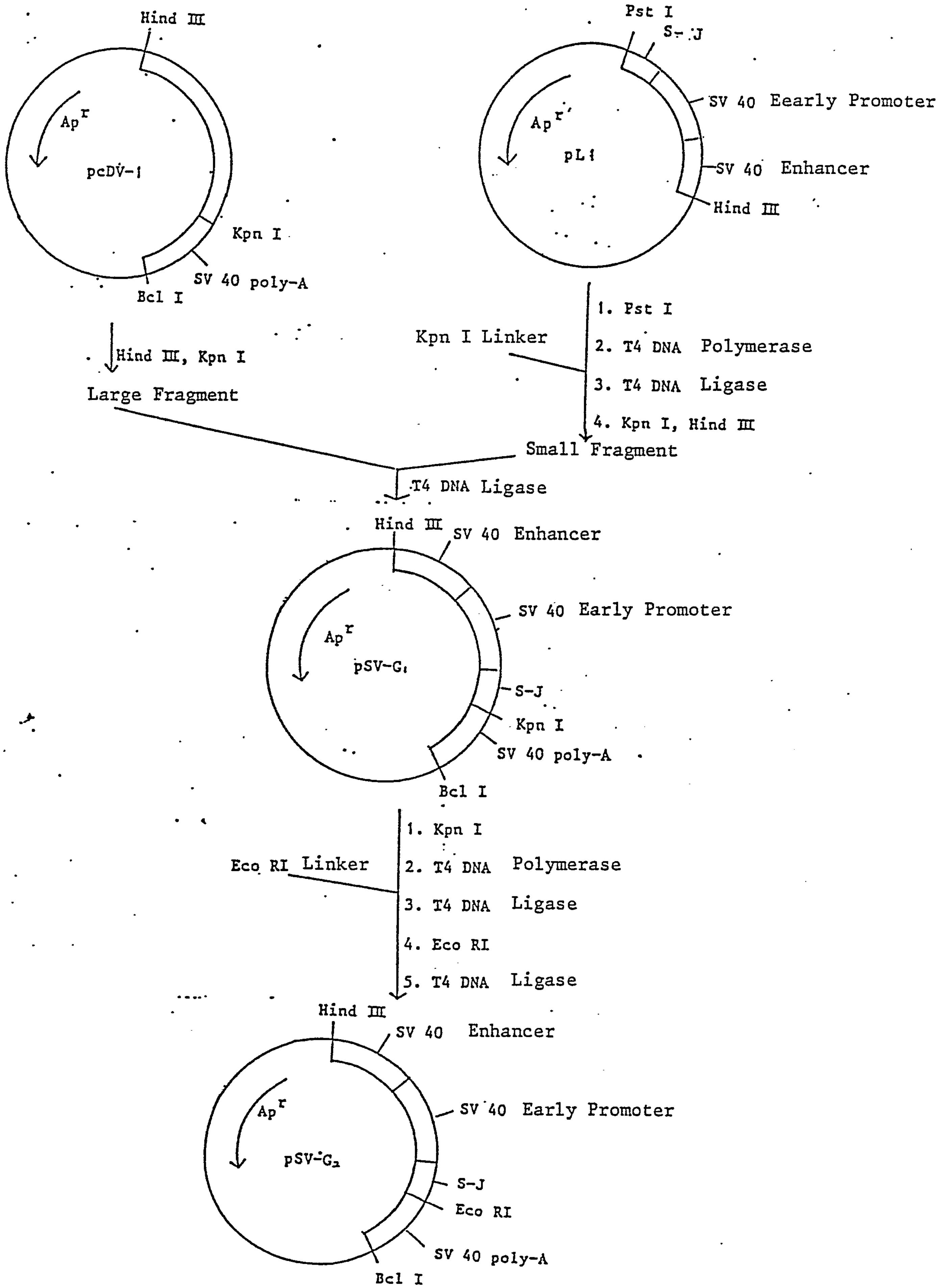

Hind III
Ap r
pcDV-1
Kpn I
SV 40 poly-A
Bcl I
Pst I
S-J
SV 40 Eearly Promoter
Ap r
pL1
SV 40 Enhancer
Hind III
Hind III, Kpn I
Large Fragment
Kpn I Linker
1. Pst I
2. T4 DNA Polymerase
3. T4 DNA Ligase
4. Kpn I, Hind III
Small Fragment
T4 DNA Ligase
Hind III
SV 40 Enhancer
SV 40 Early Promoter
Ap r
pSV-G1
S-J
Kpn I
SV 40 poly-A
Bcl I
Eco RI Linker
1. Kpn I
2. T4 DNA Polymerase
3. T4 DNA Ligase
4. Eco RI
5. T4 DNA Ligase
Hind III
SV 40 Enhancer
SV 40 Early Promoter
Ap r
pSV-G2
S-J
Eco RI
SV 40 poly-A
Bcl I

Fig. 5

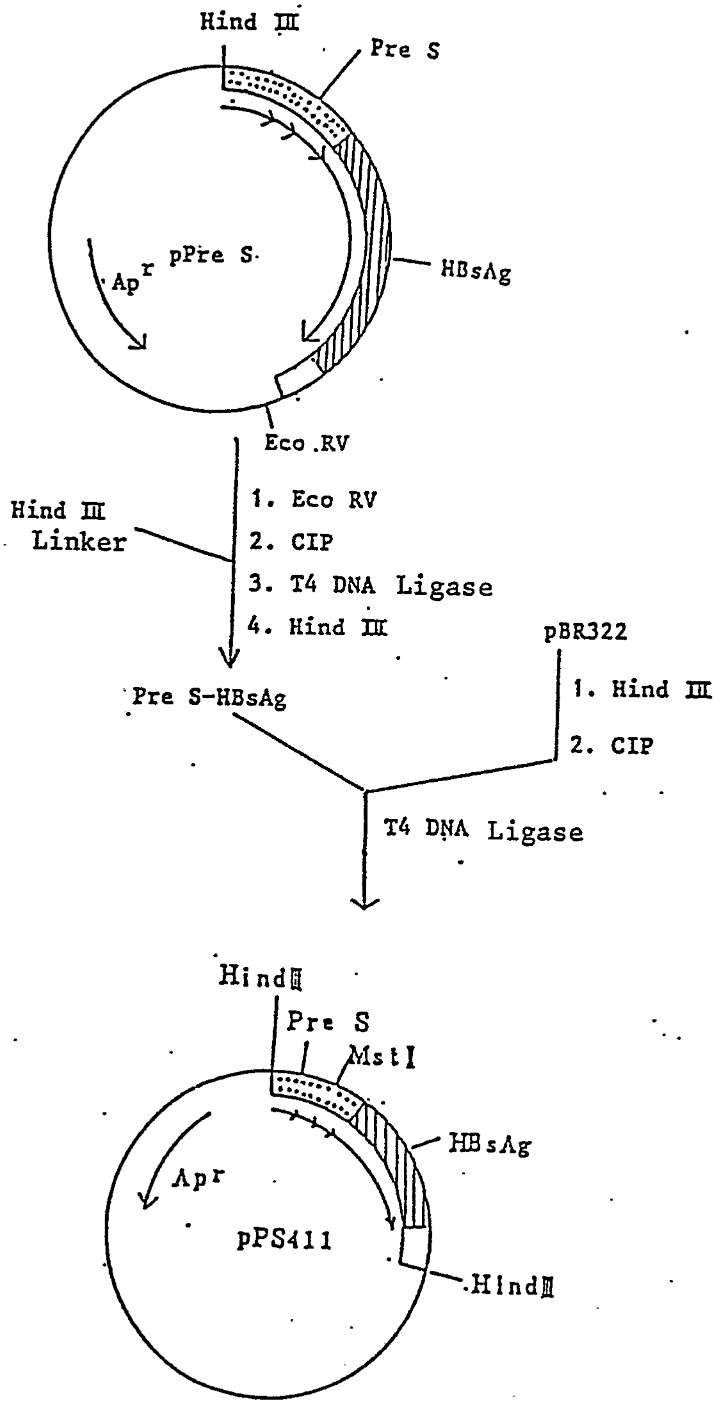
Hind III
Pre S
pPre S
Apr
HBsAg
Eco.RV
Hind III
Linker
1. Eco RV
2. CIP
3. T4 DNA Ligase
4. Hind III
pBR322
Pre S-HBsAg
1. Hind III
2. CIP
T4 DNA Ligase
HindIII
Pre S
MstI
HBsAg
Apr
pPS411
HindIII

Fig. 6

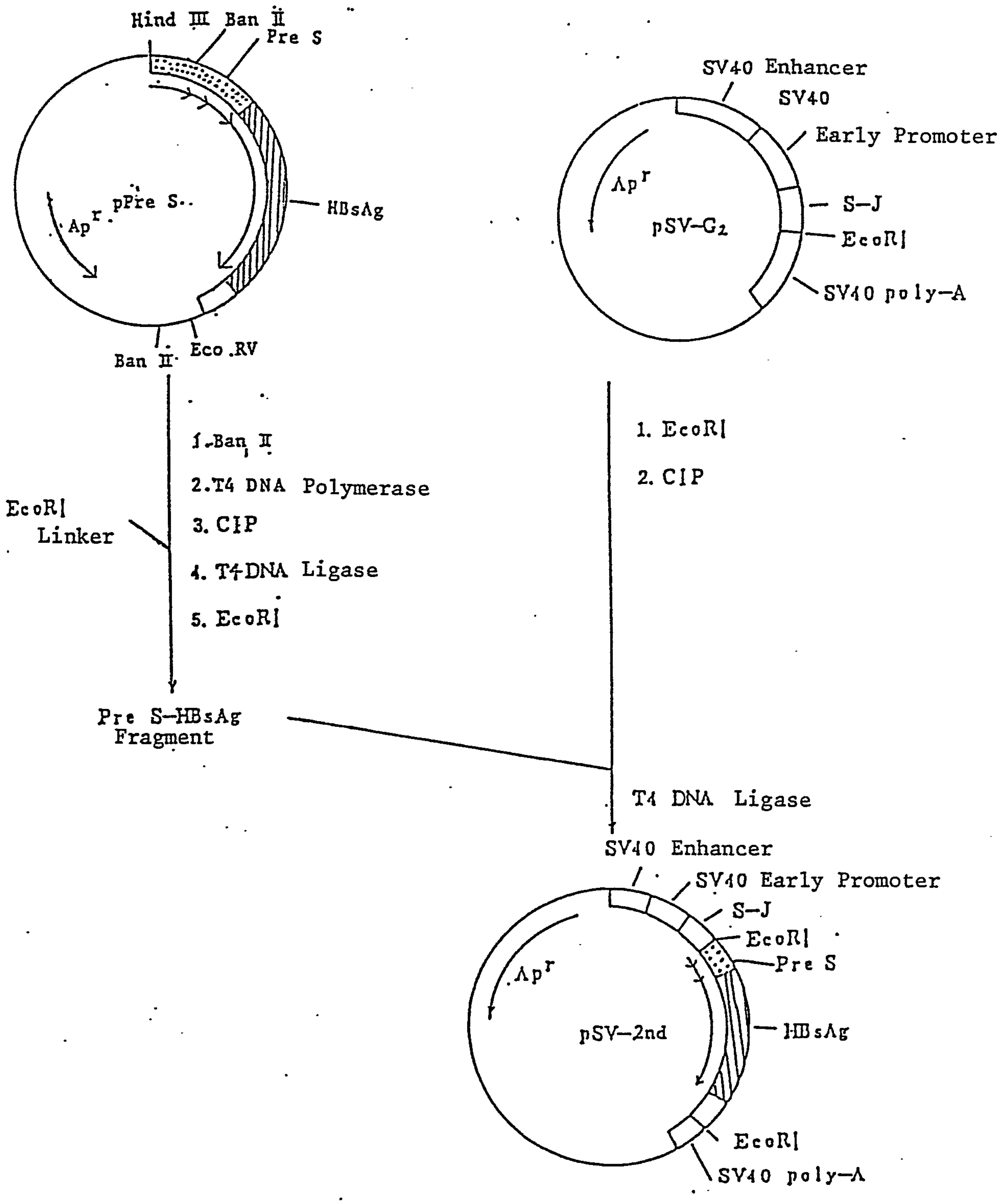
Hind III
Ban II
Pre S
HBsAg
Apr
pPre S
Ban II
Eco RV
SV40 Enhancer
SV40
Early Promoter
S-J
EcoRI
SV40 poly-A
Apr
pSV-G2
1. Ban II
2. T4 DNA Polymerase
3. CIP
4. T4 DNA Ligase
5. EcoRI
EcoRI
Linker
1. EcoRI
2. CIP
Pre S-HBsAg
Fragment
T4 DNA Ligase
SV40 Enhancer
SV40 Early Promoter
S-J
EcoRI
Pre S
HBsAg
Apr
pSV-2nd
EcoRI
SV40 poly-A

Fig. 7

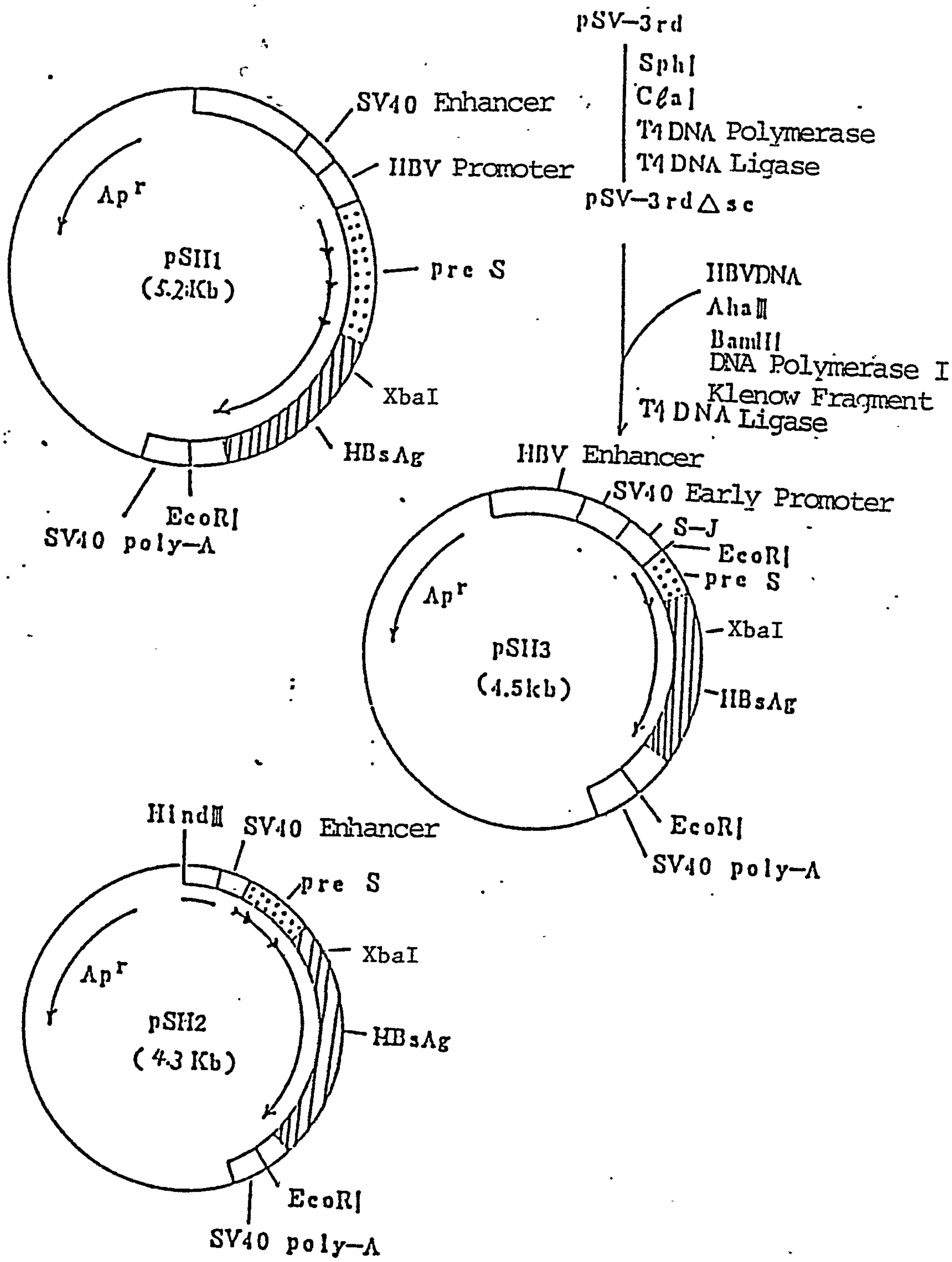
pSV-3rd
SphI
ClaI
T4 DNA Polymerase
T4 DNA Ligase
pSV-3rdΔsc
HBVDNA
AhaIII
BamHI
DNA Polymerase I
Klenow Fragment
T4 DNA Ligase
SV40 Enhancer
HBV Promoter
Apr
pSH1
(5.2Kb)
pre S
XbaI
HBsAg
EcoRI
SV40 poly-A
HBV Enhancer
SV40 Early Promoter
S-J
EcoRI
pre S
XbaI
HBsAg
pSH3
(4.5kb)
EcoRI
SV40 poly-A
HindIII
SV40 Enhancer
pre S
XbaI
Apr
pSH2
(4.3 Kb)
HBsAg
EcoRI
SV40 poly-A

Fig. 8

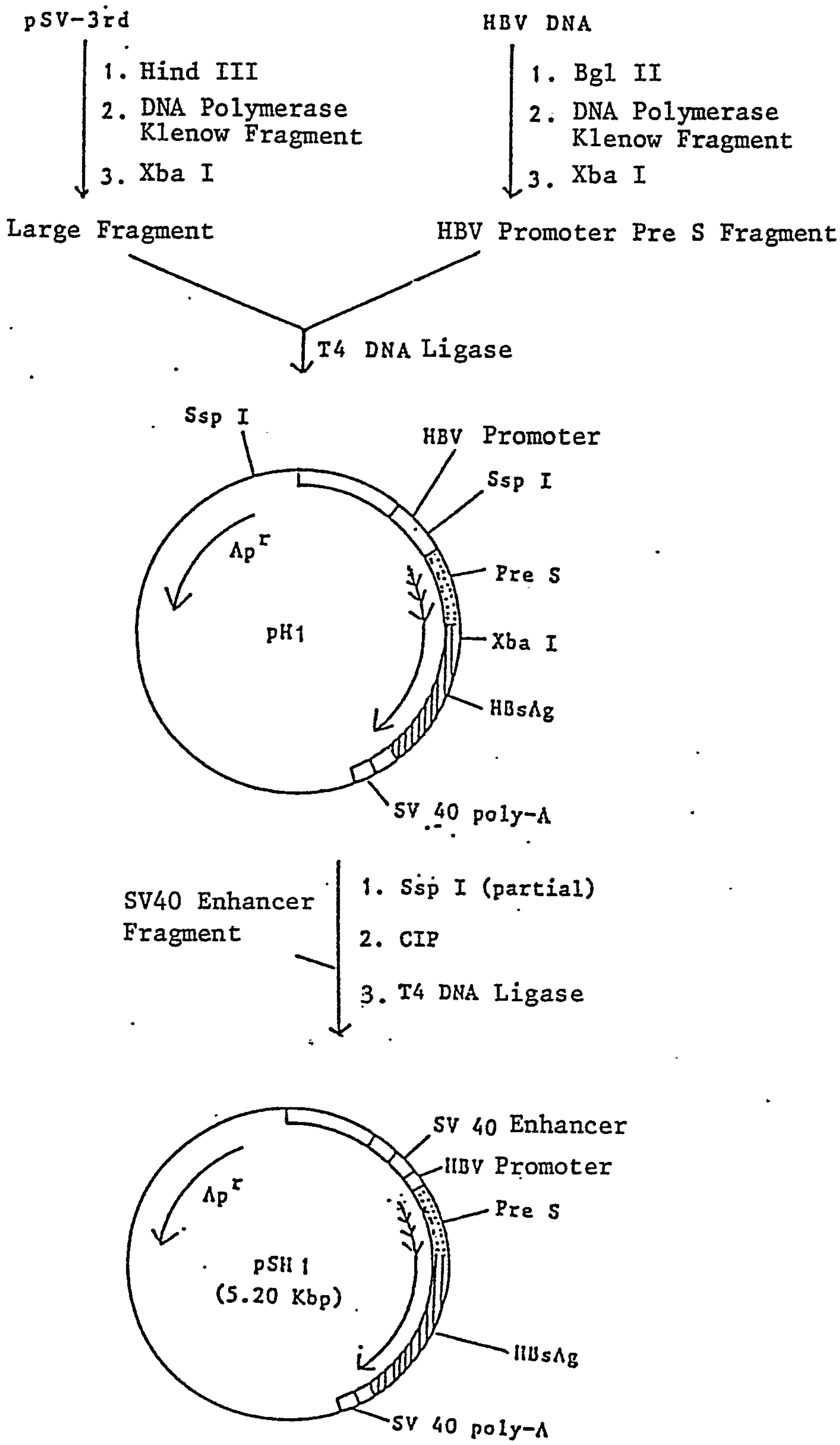
pSV-3rd
1. Hind III
2. DNA Polymerase Klenow Fragment
3. Xba I
Large Fragment
HBV DNA
1. Bgl II
2. DNA Polymerase Klenow Fragment
3. Xba I
HBV Promoter Pre S Fragment
T4 DNA Ligase
Ssp I
HBV Promoter
Ssp I
Ap^r
pH1
Pre S
Xba I
HBsAg
SV 40 poly-A
SV40 Enhancer Fragment
1. Ssp I (partial)
2. CIP
3. T4 DNA Ligase
SV 40 Enhancer
HBV Promoter
Pre S
Ap^r
pSH 1
(5.20 Kbp)
HBsAg
SV 40 poly-A

Fig. 9

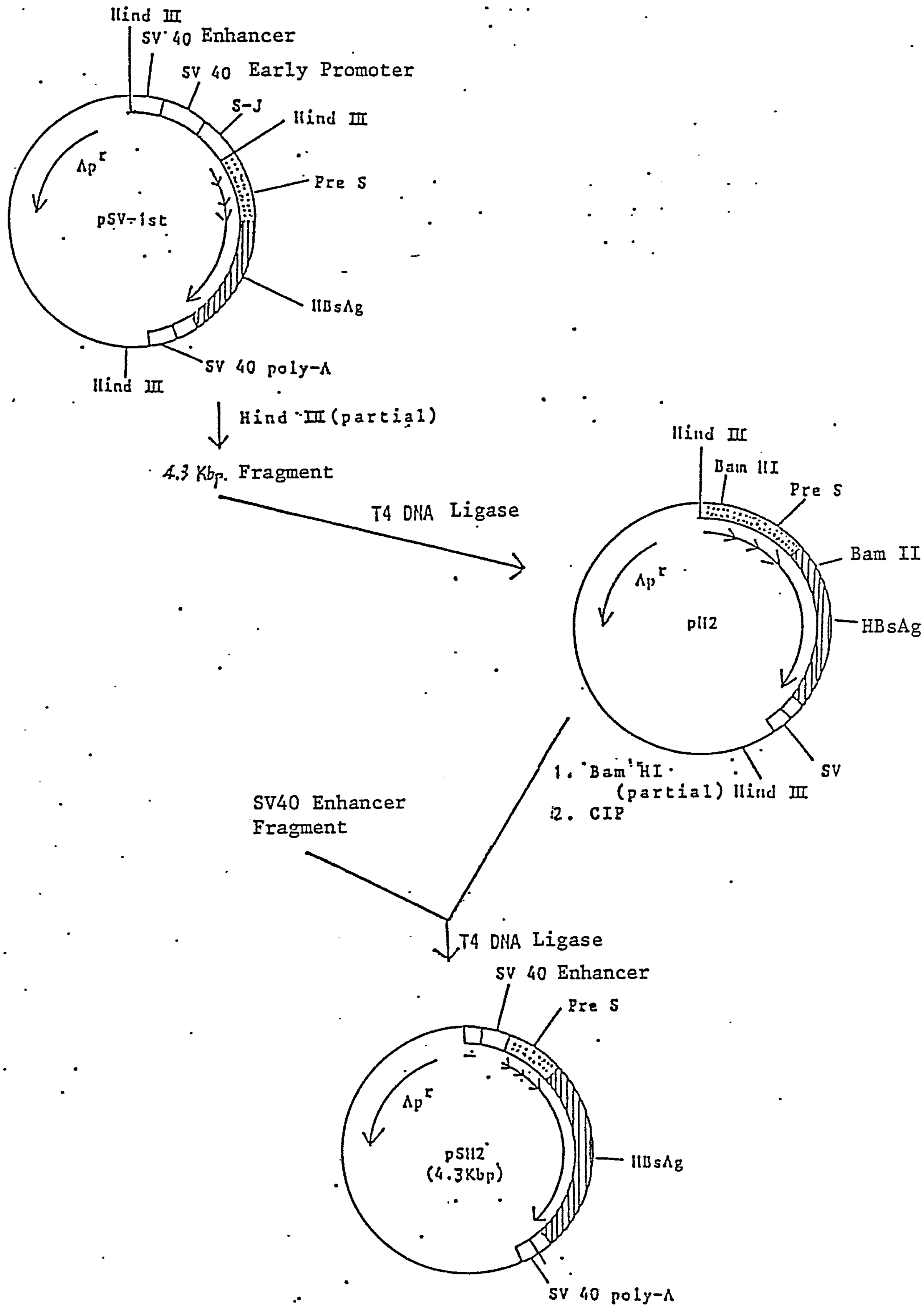
Hind III
SV 40 Enhancer
SV 40 Early Promoter
S-J
Hind III
Pre S
Ap r
pSV-1st
HBsAg
SV 40 poly-A
Hind III
Hind III (partial)
4.3 Kbp. Fragment
T4 DNA Ligase
Hind III
Bam HI
Pre S
Bam II
Ap r
pH2
HBsAg
SV
1. Bam HI (partial) Hind III
2. CIP
SV40 Enhancer Fragment
T4 DNA Ligase
SV 40 Enhancer
Pre S
Ap r
pSH2 (4.3Kbp)
HBsAg
SV 40 poly-A

Fig. 10

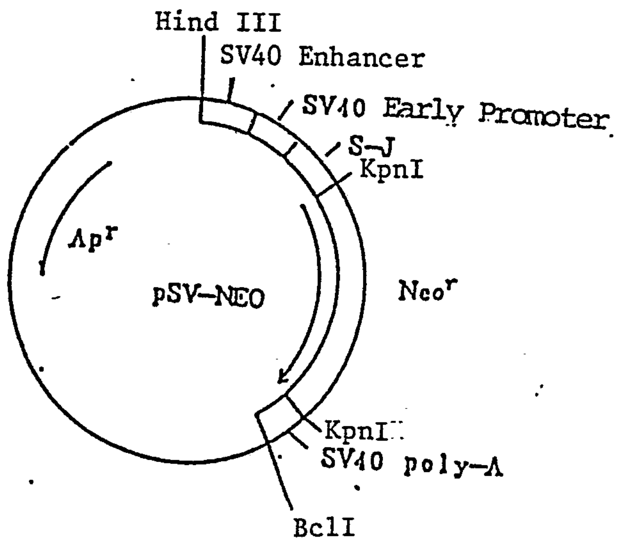
Hind III
SV40 Enhancer
SV40 Early Promoter
S-J
KpnI
Apr
pSV-NEO
Neor
KpnI
SV40 poly-A
BclI